# EUROPEAN PATENT APPLICATION

(11) **EP 3 929 086 A1**
(43) Date of publication of application: **29.12.2021**
(21) Application number: 21171986.9
(22) Date of filing: 04.05.2021
(51) Int. Cl.: B64D 11/06, B64D 13/00, B64D 13/06

(54) **AIR PURIFICATION SYSTEM**

(30) Priority: 23.06.2020 US 202063042877 P
(71) Applicant: The Boeing Company, Chicago, IL 60606-2016 (US)
(72) Inventor: Trent, Stephen M., Chicago, IL, 60606-1596 (US); Space, David R., Chicago, IL, 60606-1596 (US)
(74) Representative: Hoffmann Eitle

(57) **Abstract**

A filter assembly including a plurality of filter modules, wherein each filter module in the plurality of filter modules includes a frame, a filtration element coupled within the frame, and at least one mating feature. The at least one mating feature of each filter module is configured for selective engagement with the at least one mating feature of another filter module such that the plurality of filter modules are coupled together in a serial arrangement.

## Description

### FIELD

The field of the present disclosure relates generally to air purification systems and, more specifically, to air purification systems integrated into an aircraft, such as a passenger aircraft.

### BACKGROUND

One of the principal methods in which airborne disease spread can be transmitted is through the air inhaled, e.g., by proximity. To prevent the spread of disease, and to provide peace of mind, the passengers may be spread out within the aircraft cabin to isolate them from fellow passengers.

At least some known environmental control systems (ECS) on aircraft include recirculation systems that mix conditioned outside air bled from the compressor with cabin air and recirculate the mixed air back into the passenger cabin. Such systems use filters to capture contaminants, such as bacteria and viruses, before recirculating the air into the passenger cabin. However, known recirculation systems require a significant amount of space within the aircraft and also require a significant amount of energy to operate. Additionally, existing recirculation systems may not be sufficient to convince a passenger that they are breathing clear air.

This section is intended to introduce the reader to various aspects of art that may be related to various aspects of the present disclosure, which are described and/or claimed below. This discussion is believed to be helpful in providing the reader with background information to facilitate a better understanding of the various aspects of the present disclosure. Accordingly, it should be understood that these statements are to be read in this light, and not as admissions of prior art.

### BRIEF DESCRIPTION

In one aspect, an air purification system for purifying air in an aircraft is provided. The air purification system includes an air purification device including a filter assembly and a fan configured to draw an airflow through the filter assembly. The air purification system also includes an aircraft structure configured to support the air purification device.

In another aspect, an air craft is provided. The aircraft includes an air purification system for purifying air in the aircraft. The air purification system includes an air purification device including a filter assembly and a fan configured to draw an airflow through the filter assembly. The air purification system also includes an aircraft structure configured to support the air purification device.

In yet another aspect, a method of purifying air in proximity to a passenger seat on an aircraft is provided. The method includes supporting an air purification device using an aircraft structure, drawing an airflow into an air purification device using a fan, channeling the airflow through a filter assembly of the air purification device, and discharging the airflow in proximity to a passenger for inhalation.

Various refinements exist of the features noted in relation to the above-mentioned aspects. Further features may also be incorporated in the above-mentioned aspects as well. These refinements and additional features may exist individually or in any combination. For instance, various features discussed below in relation to any of the illustrated examples may be incorporated into any of the above-described aspects, alone or in any combination.

### BRIEF DESCRIPTION OF THE DRAWINGS

FIG. 1 is a schematic illustration of an example air purification device;
FIGS. 2A-2C are schematic illustrations of example combinations of filtration elements that may be used in the air purification device shown in FIG. 1;
FIG. 3 is a schematic illustration of an example air purification system using the air purification device shown in FIG. 1 incorporated into a sidewall assembly of an aircraft;
FIG. 4 is a schematic illustration of a second embodiment of an air purification device incorporated into a sidewall assembly of an aircraft;
FIG. 5 is a schematic illustration of another air purification system using the air purification device shown in FIG. 1 incorporated into a cabin ceiling of an aircraft;
FIG. 6 is a schematic illustration of another air purification system using the air purification device shown in FIG. 1 incorporated into a seat of an aircraft; and
FIG. 7 is a schematic illustration of a control and power scheme for the air purification device shown in FIG. 1.

Corresponding reference characters indicate corresponding parts throughout the several views of the drawings. Although specific features of various examples may be shown in some drawings and not in others, this is for convenience only. Any feature of any drawing may be referenced and/or claimed in combination with any feature of any other drawing.

### DETAILED DESCRIPTION

The air purification systems described herein include an air purification device located close to each passenger. The device can be portable or mounted in a specific location in the aircraft cabin, such as the last row of an aircraft to create a quarantine zone for sick passengers. The device can filter air with any combination of a particulate filter (such as HEPA), adsorption material, and a UV light source for disinfection. The device is located in any one of a sidewall assembly, ceiling panel, under-seat storage, in an empty seat, or floor panel. If there is a sick passenger on the aircraft, air purification system filters and disinfects the exhaled air and returns it to the cabin. The air purification device can be powered by the aircraft system or may include an internal power source. The device can be controlled via either the flight deck, attendant panels, passenger in-flight entertainment panel, or a control panel on the device itself to operate the fan and applicable filtration elements.

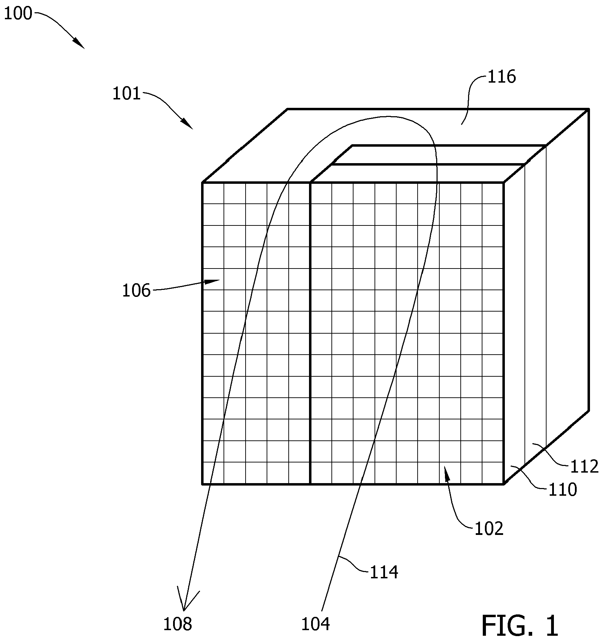

FIG. 1 is a schematic illustration of an example air purification system 100 having an air purification device 101. In the example implementation, air purification system 100 includes an inlet 102 for receiving airflow from a source 104, and an outlet 106 for discharging airflow to a predetermined location 108 (e.g., a room, a passenger cabin, or a compartment). Air purification system 100 includes a filter assembly 110 and a fan 112 positioned between inlet 102 and outlet 106, as will be described in more detail below. In operation, fan 112 draws an airflow 114 from source 104, through filter assembly 110 and through a plenum 116 of air purification system 100 before discharging airflow 114 toward location 108. More specifically, airflow 114 is channeled through filter assembly 110 before being channeled towards location 108 to facilitate removing contaminants from the air channeled through air purification system 100 from source 104. For example, the contaminants may be in either solid particulate, such as dust, pollen, mold, bacteria, and viruses, or gaseous form. Filter assembly 110 includes any number of filtration elements that enables air purification system 100 to function as described herein.

In a particular implementation, air purification system 100 is included onboard an aircraft (not shown), for example, and is independent of an environmental control system (ECS) of the aircraft. More specifically, air purification device 101 operates in conjunction with ECS to provide additional purification of the air within the passenger cabin or crew area. In such an implementation, source 104 and location 108 are both a passenger cabin, are both a cockpit, and are both a crew compartment, for example. Moreover, while air purification system 100 is capable of use within an aircraft, air purification system 100 can also be implemented in any structure through which air flows, such as a building, a platform, or a vehicle. Further, air purification device 101 is capable of utilization in any system where filtering contaminants from a stream of air is desired.

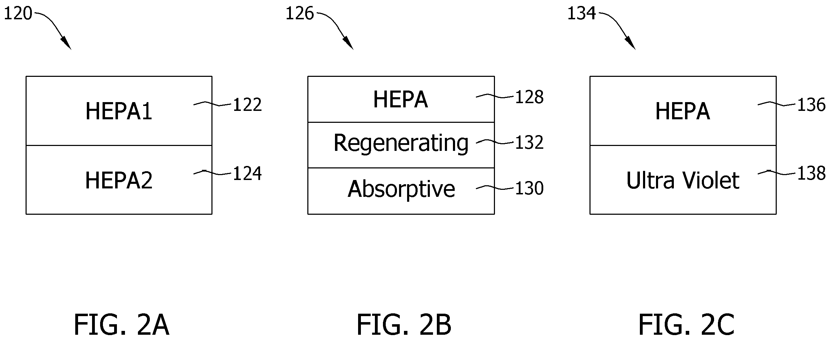

FIGS. 2A-2C are schematic illustrations of example combinations of filtration elements that may be used in filter assembly 110 of air purification device 101. Example filtration elements coupled within filter assembly 110 include, but are not limited to, a particulate filter media (i.e., high-efficiency particulate air (HEPA) filter media), an absorptive filter media, and functional filtration devices, such as a regenerative heating element, an ultraviolet irradiation element, and/or an ozone converter element. The particulate filter media facilitates removing particulate contaminants from airflow 114 (shown in FIG. 1). More specifically, "high efficiency particulate arresting" HEPA filters, trap more than 99% (Mil Std 282) of all bacteria and clustered viruses. HEPA filters remove essentially all airborne pathogens and other particulate matter from an airstream that passes through them with a minimal efficiency of 99.97% for 0.3-µm particles.

The absorptive filter media, such as but not limited to, activated charcoal, facilitates removing gaseous contaminants from airflow 114. These filters are available as an option on some aircraft to adsorb organic gases that are not trapped by the HEPA filters. The regenerative heating element is capable of regenerating the absorptive filter media. The ultraviolet irradiation element facilitates neutralizing viruses and bacteria entrained in airflow 114, and the ozone converter element is a catalytic converter device that facilitates generating oxygen from ozone-rich ambient air, for example.

As shown in FIGs 2A-2C, the plurality of filtration elements in filter assembly 110 are coupled together in predetermined combinations or series based on a type of filtration element coupled within each filtration element. Moreover, when filter assembly 110 includes a particulate filter media and an absorptive filter media, the particulate filter media is positioned upstream from the absorptive filter media. As such, the particulate filter media removes particulate contaminants from airflow 114 to reduce fouling of the absorptive filter media. Similarly, when filter assembly 110 includes an absorptive filter media and a regenerative heating element, the regenerative heating element is positioned upstream from the absorptive filter media. As such, airflow 114 is heated before being channeled through absorptive filter media, which facilitates regenerating the absorptive material contained within absorptive filter media.

Referring to FIG. 2A, for illustrative purposes, a first filter assembly combination 120 includes a first HEPA module 122 and a second HEPA module 124 coupled together in a predetermined series. In some implementations, first HEPA module 122 and second HEPA module 124 have different filtration capabilities, thereby increasing the filtration efficiency of the overall assembly.

Referring to FIG. 2B, a second filter assembly combination 126 includes a HEPA module 128 and an absorptive module 130 coupled together in a predetermined series. An optional regenerating unit 132 (represented by dashed lines) is positioned between HEPA module 128 and absorptive module 130.

Referring to FIG. 2C, a third filter assembly combination 134 includes HEPA module 136 and an ultra violet light unit 138 coupled together in a predetermined series.

The filter assemblies shown in FIGs 2A-2C are for example purposes only, and any filter assembly 110 including different filtration elements, or more than one of the same filtration element, or only a single one of any of the filtration elements described herein can be assembled as described herein. For example, even though FIGs 2A-2C illustrate each filter assembly combination as including multiple filtration elements, filter assembly 110 may include only one of the filtration elements described herein.

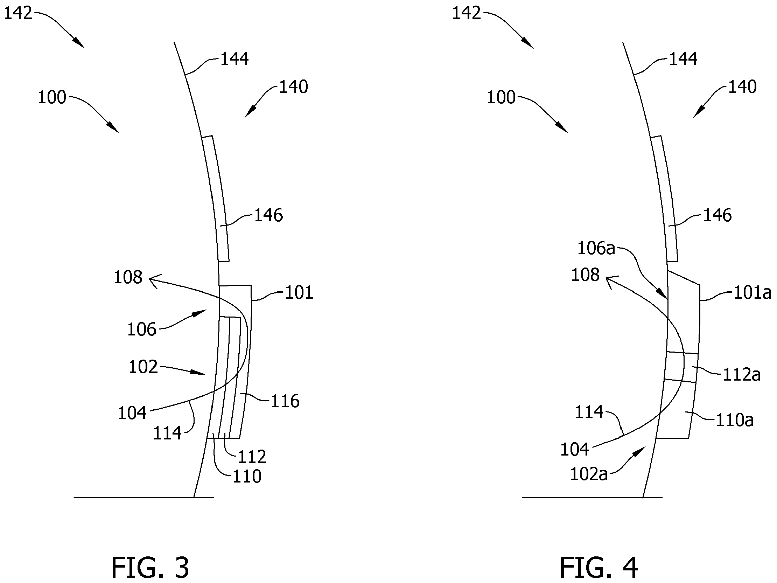

FIG. 3 is a schematic illustration of an example air purification system 100 using air purification device 101 incorporated into a sidewall assembly 140 of an aircraft 142. Sidewall assembly 140 includes a sidewall 144 and a window 146. Sidewall 144 includes inlets and outlets that correspond to inlet 102 and outlet 106 to enable airflow 114 therethrough to facilitate operation of air purification device 101. Air purification device 101 is attached to sidewall 144 such that inlet 102 is positioned lower on sidewall 144 toward a passenger's feet, and outlet 106 is positioned toward window 146. In such a configuration, airflow 114 is taken from a source 104 where recently exhaled breath is not present and discharge location 108 is positioned such that airflow 114 exiting outlet 106 is channeled towards a passenger's face. As such, the passenger is provided with air that has immediately previously undergone filtration to remove airborne contaminates.

FIG. 4 is a schematic illustration of a second air purification system 100a using an air purification device 101a incorporated into sidewall assembly 140 of aircraft 142. In the illustrated embodiment, air purification device 101a includes the same components as air purification device 101, but in a different configuration. Instead of an L-shaped plenum 116, air purification device 101a includes a plenum 116a that does not channel the airflow 114 around a 90 degree angle. Additionally, air purification device 101a may include a different filtration element than device 101 and so may require a different configuration. Sidewall 144 includes inlets and outlets that correspond to inlet 102a and outlet 106a to enable airflow 114 therethrough to facilitate operation of air purification device 101a. Air purification device 101a is attached to sidewall 144 such that inlet 102a is positioned lower on sidewall 144 toward a passenger's feet, and outlet 106a is positioned toward window 146. In such a configuration, airflow 114 is taken from a source 104 where recently exhaled breath is not present and discharge location 108 is positioned such that airflow 114 exiting outlet 106a is channeled towards a passenger's face. As such, the passenger is provided with air that has immediately previously undergone filtration to remove airborne contaminates.

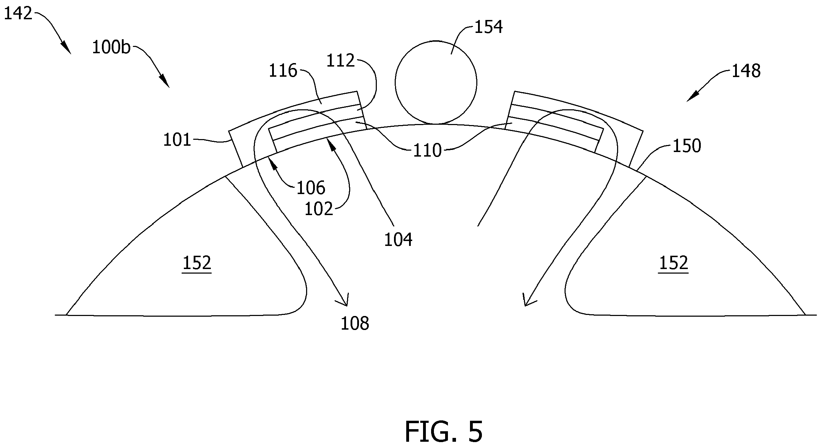

FIG. 5 is a schematic illustration of another air purification system 100b using air purification devices 101 incorporated into a cabin ceiling assembly 148 of aircraft 142. In the illustrated embodiment, cabin ceiling assembly 148 includes a ceiling panel 150, a pair of opposing storage bins 152, and an air distribution duct 154 of the ECS. Ceiling panel 150 includes inlets and outlets that correspond to inlet 102 and outlet 106 to enable airflow 114 therethrough to facilitate operation of air purification device 101. Air purification device 101 is attached to ceiling panel 150 such that inlet 102 is positioned toward a center of an aisle area 156, and outlet 106 is positioned toward storage bins 152. In such a configuration, airflow 114 is taken from a source 104 where recently exhaled breath is not present and discharge location 108 is positioned such that airflow 114 exiting outlet 106 is channeled down towards a passenger's face. As such, the passenger is provided with air that has immediately previously undergone filtration to remove airborne contaminates.

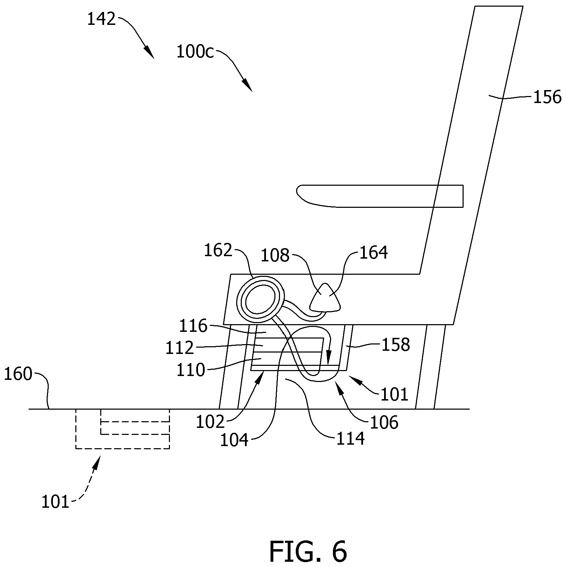

FIG. 6 is a schematic illustration of another air purification system 100c using the air purification device 101 incorporated into a seat 156 of aircraft 142. In one embodiment, aircraft 142 includes multiple air purification devices 101, which may be in any location, such as in any combination of systems 100, 100a, 100b, and 100c shown in FIGs 3-6. In the embodiment shown in FIG. 6, air purification device 101 is positioned on a rack 158 located beneath seat 156. Alternatively, or in combination, air purification device 101 (shown as optional in broken lines) may be located beneath a floor panel 160 of aircraft 142. Alternatively, or in combination, air purification device 101 may be located on an empty adjacent seat 156 or on the rear of the seat in front. Generally, air purification device 101 may be located anywhere in aircraft 142 that facilitates operation of systems 100, 100a, 100b, and 100c as described herein. Furthermore, air purification device 101 is portable to facilitate locating air purification device 101 in various locations on aircraft 124 as needed.

As shown in FIG. 6, air purification system 100c includes a length of tubing 162 coupled in flow communication with outlet 106 and a mask 164 coupled in flow communication with tubing 162. In operation, the passenger removes mask 164 from a storage location (not shown) and wears mask 164 on their face to cover their nose and mouth. In such a configuration, airflow 114 is taken from a source 104, beneath seat 156, where recently exhaled breath is not present and mask 164 serves as discharge location 108 such that airflow 114 exiting outlet 106 is channeled through tubing 162 directly to the passenger. As such, the passenger is provided with air that has immediately previously undergone filtration to remove airborne contaminates without being exposed to nonfilter air within the aircraft cabin.

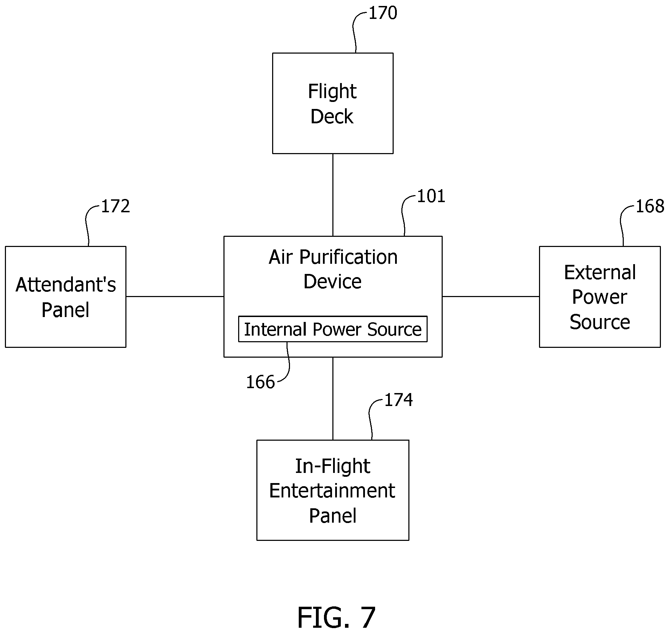

FIG. 7 is a schematic illustration of a control and power scheme for the air purification device 101. In one implementation, air purification device 101 includes an internal power source 166, such as a battery, that provides electric power to fan 112 and to filter assembly 110, as needed. For example, in embodiments where filter assembly 110 includes a regeneration unit or an ultra violet light unit, internal power source 166 provides power to facilitate operation thereof. Alternatively, or in combination, air purification device 101 is electrically connected to an external power source 168. In such an embodiment, air purification device 101 may be plugged into an electric outlet (not shown) on aircraft 142. Generally, air purification device 101 receives power in any way that facilitates operation as described herein.

In one embodiment, operation of air purification device 101 is suitably controlled by the pilot or co-pilot from the flight deck 170. Alternatively, or in combination, operation of air purification device 101 is suitably controlled from a flight attendant's panel 172 located in the aircraft cabin. Alternatively, or in combination, operation of air purification device 101 is controlled by the passenger, e.g., from the in-flight entertainment panel 174. Generally, operation of air purification device 101 may be controlled from any location that enables the operator to control air purification device 101 to operate fan 112 and any applicable filtration elements of filter assembly. Because aircraft 124 includes multiple air purification devices 101, in embodiments where air purification devices 101 are controlled from the flight deck 170 or the attendant's panel, the pilot or flight attendant can control each air purification device individually.

The air purification systems described herein include an air purification device located close to each passenger. The device can be portable or mounted in a specific location in the aircraft cabin, such as the last row of an aircraft to create a quarantine zone for sick passengers. The device can filter air with any combination of a particulate filter (such as HEPA), adsorption material, and a UV light source for disinfection. The device is located in any one of a sidewall assembly, ceiling panel, under-seat storage, in an empty seat, or floor panel. If there is a sick passenger on the aircraft, air purification system filters and disinfects the exhaled air and returns it to the cabin. The air purification device can be powered by the aircraft system or may include an internal power source. The device can be controlled via either the flight deck, attendant panels, passenger in-flight entertainment panel, or a control panel on the device itself to operate the fan and applicable filtration elements. The air purification device can also be used when a passenger is quarantined away from other passengers to help prevent the spread of infectious diseases. Such as a separate seat in the back of the plane, the crew rest or attendant rest in the aircraft.

The systems and methods described are not limited to the specific embodiments described herein, but rather, components of the systems and/or steps of the methods may be utilized independently and separately from other components and/or steps described herein.

Although specific features of various embodiments of the disclosure may be shown in some drawings and not in others, this is for convenience only. In accordance with the principles of the disclosure, any feature of a drawing may be referenced and/or claimed in combination with any feature of any other drawing.

As used herein, an element or step recited in the singular and proceeded with the word "a" or "an" should be understood as not excluding plural elements or steps unless such exclusion is explicitly recited. Furthermore, references to "one embodiment" of the present invention or the "example embodiment" are not intended to be interpreted as excluding the existence of additional embodiments that also incorporate the recited features.

This written description uses examples to disclose various implementations, including the best mode, and also to enable any person skilled in the art to practice the various implementations, including making and using any devices or systems and performing any incorporated methods. The patentable scope of the disclosure is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. An air purification system for purifying air in an aircraft, the air purification system comprising:
an air purification device comprising a filter assembly and a fan configured to draw an airflow through the filter assembly; and
an aircraft structure configured to support the air purification device.

2. The air purification system in accordance with Claim 1, wherein the aircraft structure comprises at least one of a sidewall assembly, a ceiling panel, a passenger seat, and a floor panel.

3. The air purification system in accordance with any preceding claim, wherein the air purification device is portable about a cabin of the aircraft.

4. The air purification system in accordance with any preceding claim, wherein the air purification device is remotely controlled from at least one of a flight deck, an attendant's panel, and an in-flight entertainment panel.

5. The air purification system in accordance with any preceding claim, wherein the air purification device comprises an internal power source.

6. The air purification system in accordance with any preceding claim, wherein the air purification device is powered by the aircraft.

7. The air purification system in accordance with any preceding claim, wherein the air purification device comprises an inlet located away from exhaled breath, and an outlet located to channel filtered air toward a passenger's face.

8. The air purification system in accordance with any preceding claim, wherein the filter assembly comprises at least one of a particulate filter, an adsorption material, and an ultra violet light source.

9. The air purification system in accordance with any preceding claim, further comprising tubing and a mask coupled in flow communication with an outlet of the air purification device for directly supplying disinfected air to a passenger.

10. The air purification system in accordance with any preceding claim, wherein the air purification device is configured to disinfect the air a passenger breathes in.

11. The air purification system in accordance with any preceding claim, wherein the air purification device is configured to disinfect the air a passenger exhales.

12. The air purification system in accordance with any preceding claim, wherein the air purification device is configured to remove gaseous compounds

13. An aircraft comprising:
an air purification system for purifying air in the aircraft, the air purification system comprising:
an air purification device comprising a filter assembly and a fan configured to draw an airflow through the filter assembly; and
an aircraft structure configured to support the air purification device.

14. A method of purifying air in proximity to a passenger seat on an aircraft, the method comprising:
supporting an air purification device using an aircraft structure;
drawing an airflow into an air purification device using a fan;
channeling the airflow through a filter assembly of the air purification device; and
discharging the airflow in proximity to a passenger for inhalation.
